Europäisches Patentamt

European Patent Office

Office européen des brevets

(11) Publication number: **0 276 551 B1**

## EUROPEAN PATENT SPECIFICATION

(45) Date of publication of patent specification: **18.03.92**

(51) Int. Cl.⁵: **C07K 13/00**, C07K 3/02, C07K 3/20, A61K 37/02

(21) Application number: **87310739.5**

(22) Date of filing: **07.12.87**

(54) **Colony-stimulating factor and method for preparation thereof.**

(30) Priority: **07.12.86 JP 291294/86**
**06.04.87 JP 83037/87**
**17.07.87 JP 178697/87**

(43) Date of publication of application:
**03.08.88 Bulletin 88/31**

(45) Publication of the grant of the patent:
**18.03.92 Bulletin 92/12**

(84) Designated Contracting States:
**AT CH DE FR GB LI**

(56) References cited:
**WO-A-86/04587**
**US-A- 4 275 056**
**US-A- 4 504 586**

**EXP. HEMAT, vol. 12, 1984, page 434, abstract no. 195; A. WAHEED et al.: "A rapid technique for the purification of human urinary CSF"**

(73) Proprietor: **MORINAGA MILK INDUSTRY CO., LTD.**
**33-1, Shiba 5-chome**
**Minato-ku, Tokyo 108(JP)**

(72) Inventor: **Takaku, Fumimaro**
**43-30, Asahigaoka-1-chome**
**Nerima-ku Tokyo(JP)**
Inventor: **Motoyoshi, Kazuo**
**1238, Kamikocho**
**Omiya-shi(JP)**
Inventor: **Kawashima, Takuji**
**18-5, Tsuchihashi-3-chome**
**Miyamae-ku Kawasaki-shi(JP)**
Inventor: **Saito, Minoru**
**20-9, Higashinogawa-4-chome**
**Komae-shi(JP)**
Inventor: **Yanai, Nobuya Morinaga Nyugyo K.K.Nakano**
**Shataku 401 21-10, Minamidai-5-chome**
**Nakano-ku Tokyo(JP)**
Inventor: **Yamada, Muneo**
**Sakae Terasu 205 752, Kuji**
**Takatsu-ku Kawasaki-shi(JP)**

Rank Xerox (UK) Business Services

EP 0 276 551 B1

CHEMICAL ABSTRACTS, vol. 9, no. 9, 30th August 1982, page 486, abstract no. 70635n, Columbus, Ohio, US; A. WAHEED et al.: "Purification of colony-stimulating factor by affinity chromatography", & BLOOD 1982, 60(1), 238-44

CHEMICAL ABSTRACTS, vol. 100, no. 7, 13th February 1984, page 62, abstract no. 45417r, Columbus, Ohio, US; F.F. WANG et al.: "Purification of a human urinary colony-stimulating factor", & J. CELL. BIOCHEM. 1983, 21(4), 263-75

CHEMICAL ABSTRACTS, vol. 103, no. 25, 23rd December 1979, pa ge 696, abstract no. 212902r, Columbus, Ohio, US; K. HATAKE et al.: "Purification of human urinary colony-stimulating factor by high-performance liquid chromatography", & J. CHROMATOGR. 1985, 344, 339-44

CHEMICAL ABSTRACTS, vol. 90, no. 1, 1st January 1979, page 192, abstract no. 1931z, Columbus, Ohio, US; K. MOTOYOSHI et al: "Purification and some properties of colony-stimulating factor from normal human urine", & BLOOD 1978, 52(5), 1012-20

CHEMICAL ABSTRACTS, vol. 99, no. 1, 4th July 1983, page 116, abstract no. 1046a, Columbus, Ohio, US; K. MOTOYOSHI et al.: "Phase I and early phase II studies on human urinary colony stimulating factor", & JPN. J. MED. 1982, 21(3), 187-91

THE JOURNAL OF BIOLOGICAL CHEMISTRY, vol. 257, no. 22, 25th November 1982, pages 13679-13684, US; S.K. DAS et al.: "Structure-function studies of a colony stimulating factor (CSF-1)"

BIOCHEMISTRY, vol. 17, no. 15, 1978, pages 3109-3115; S.S. FOJO et al.: "Purification and characterization of a colony stimulating factor from human lung"

WO 87/06954 G. WONG et al. 1987, Science, 235: 1504-1508

Inventor: Yokota,Hajime
5-1-406, Shirokane-2-chome
Minato-ku Tokyo(JP)
Inventor: Ujiie, Kunio Morinaga Nyugyo K.K. Ikegamiryo
10-16, Nakaikegami-2-chome
Ota-ku Tokyo(JP)

(74) Representative: Harrison, David Christopher et al
MEWBURN ELLIS & CO 2/3 Cursitor Street
London EC4A 1BO(GB)

2

## Description

This invention relates to a novel colony-stimulating factor which can be isolated from human urine and which stimulates the colony formation of monocyte-macrophage cells of mammals and to methods for their preparation from media conditioned by colony stimulating factor producing cells and colony stimulating factor gene recombined cells.

Colony-stimulating factor (referred to as "CSF" hereinafter) is a hematopoietic factor which stimulates differentiation and proliferation of hematopoietic stem cells present in hematopoietic tissues such as bone marrow of mammals, and most of CSF comprises glycoprotein. Hitherto, there have been four factors, namely, a factor which acts on monocyte-macrophage stem cells (M-CSF or CSF-1), a factor which acts on granulocyte-monocyte stem cells (GM-CSF), a factor which acts on granulocyte stem cells (G-CSF) and a factor which acts on multipotent stem cells which are common to granulocytes, monocytes, erythrocytes and megakaryocytes (Multi-CSF, interleukin-3 or IL-3).

With reference to three of the above human origin CSF (ie except for Multi-CSF), gene cDNAs which code for respective amino acid sequences are cloned and their protein structures have been elucidated [cf. G.G. Wong et al, Science, vol. 228, pages 810-815, 1985; E.S. Kawasaki et al, Science, vol. 230, pages 291-296, 1985; S. Nagata et al, Nature, vol. 319, pages 415-418, 1986].

As CSF present in human urine, there have been reported a factor which acts on monocyte-macrophage cells (CSF-1 or M-CSF) [S.K. Das & E.R. Stanley, Journal of Biological Chemistry, vol. 257, pages 13679-13684, 1982], an HGI-glycoprotein which stimulates granulocyte colony [Japanese Patent Examined Publication (Kokoku) No. 30291/85 corresponding to United States Patent No. 4275056] and CSF-HU [K. Motoyoshi et al. Blood. vol. 52, pages 1012-1020, 1978 and Blood, vol. 60, pages 1378-1386, 1982].

Kawasaki et al, supra, describe the complete purification of such a CSF-1 obtained from human urine and which acts on monocyte-macrophage cells. This purification is achieved by an immunoaffinity chromatography using a rat monoclonal antibody to murine CSF-1.

In addition, Kawasaki et al also describe the preparation, from the pancreatic tumour cell line MIAPaCa-2, of a cDNA which codes for human CSF-1, expression of the cDNA clone, and sequencing of the resulting protein.

The structure of the above purified CSF-1 obtained from human urine is such that two polypeptides each containing a sugar chain form a biologically active homodimer through disulfide bonding. This dimer forms the same two subunits by breaking of the disulfide bond with a reducing agent. The molecular weight of this biologically active glycoprotein measured by the sodium dodecyl sulfate polyacrylamide electrophoresis method is 45,000 - 60,000 daltons.

The number of amino acids predicted by cDNA encoding the amino acid sequence of the CSF-1 subunit without glycosylation is 224, which leads to a theoretically predicted molecular weight of 26,000 daltons. However, it is found that the molecular weight of the polypeptide subunit without glycosylation is in fact, 14,000 - 17,000 daltons, from which it is assumed that the subunits are proteolytically processed to allow release from the producing cells.

WO-A-8604587 also discloses the purification of human CSF-1 proteins derived from human urine and from MIAPaCa cells. In agreement with Kawasaki et al, supra, mature human urinary CSF-1 is said to be a highly glycosylated dimer of 45kd and is also isolated, after standard partial purification techniques, using a rat monoclonal antibody to murine CSF-1.

US-A-4504586 describes the isolation of CSF-1 from biological fluids by affinity purification using a monoclonal antibody to CSF-1 isolated from a human pancreatic carcinoma cell line , ie the abovementioned MIAPaCa, so as to obtain a MIAPaCa CSF-1.

We have found now that human urine also contains another glycoprotein CSF not previously found in human urine, which glycoprotein CSF is physicochemically distinguishable from the abovementioned CSF-1 and have isolated it and elucidated its physicochemical and biological properties. As a result, this invention has been accomplished.

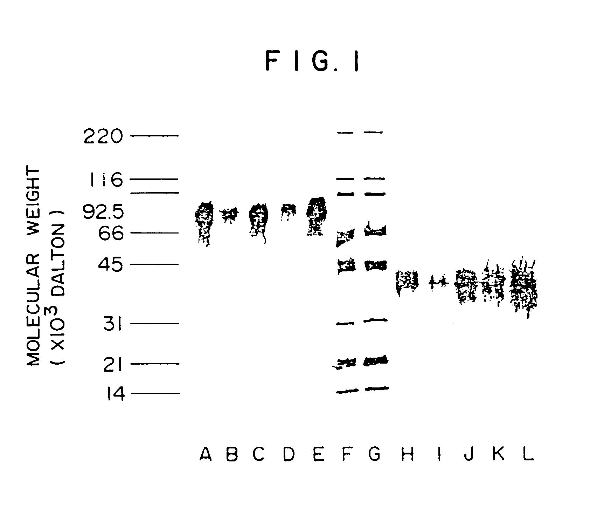

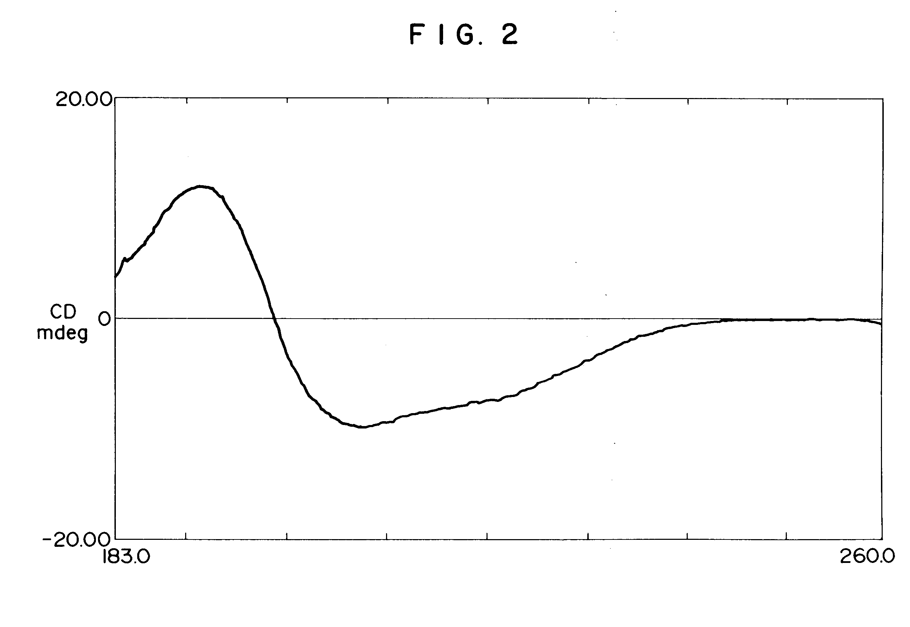

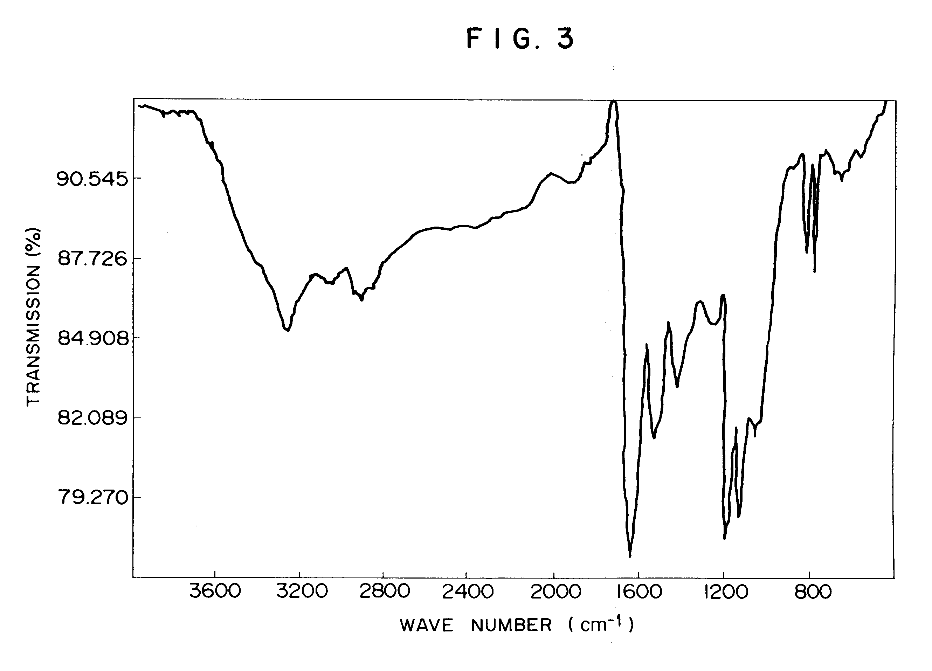

Fig. 1 is a diagram of sodium dodecyl sulfate polyacrylamide gel electrophoresis (SDS-PAGE) of the CSF-1 isolated by the method of this invention and Figs. 2 and 3 show far ultraviolet CD spectrum and Infared spectrum of the CSF of this invention, respectively.

The invention provides a method of isolating a CSF as hereinafter defined from human urine. Such a method may result in the isolation of a glycoprotein having subunits each compound of 214 amino acids and not previously disclosed in the literature and the invention also provides such a glycoprotein and a pharmaceutical composition comprising the CSF and a pharmaceutical acceptable adjuvant. The CSF isolated by the method of the invention may be used to provide a therapeutic effect in the treatment of leukopenia, which may be caused, for example, by chemotherapy with administration of anticancer drugs,

3

immuno-deficiency or transplantation of bone marrow.

That is, this invention provides a method of isolating, from human urine, a CSF comprising a glycoprotein having the following physicochemical properties and having the stimulating action of the formation of a colony of monocyte-macrophage cells of mammals.

(a) Molecular weight:

It is a homodimer which can be dissociated into two subunits containing the same respective amino acid sequences with a reducing agent in the same manner as for CSF-1 and the molecular weight of the glycoprotein measured by sodium dodecyl sulfate polyacrylamide gel electrophoresis is 70,000 - 90,000 daltons. The molecular weight of the glyco-polypeptide subunit (which has been dissociated with a reducing agent and has lost its biological activity) measured by sodium dodecyl sulfate polyacrylamide gel electrophoresis is 35,000 - 45,000 daltons.

(b) Amino acid sequence of subunit:

The glycopolypeptide subunit of the homodimer contains an amino acid sequence of 189, 214 or 238 amino acids as shown below and the 122nd and 140th asparagines (Asn) may have N-glycosidic linkage sites represented by asparagine (Asn)-X-threonine (Thr)/serine (Ser), respectively. X represents an optional amino acid.

EP 0 276 551 B1

1
Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

50
-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

100
-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

122                                                                                                                 140
-Phe-Asn-Glu-Thr-Lys-Asn-Leu-Leu-Asp-Lys-Asp-Trp-Asn-Ile-Phe-Ser-Lys-Asn-Cys-Asn-

150
-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

189
-Pro-Ser-Met-Ala-Pro-Val-Ala-Gly-Leu-


Amino acid sequence of subunit having 189 amino acids

Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

-Phe-Asn-Glu-Thr-Lys-Asn-Leu-Leu-Asp-Lys-Asp-Trp-Asn-Ile-Phe-Ser-Lys-Asn-Cys-Asn-

-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

-Pro-Ser-Met-Ala-Pro-Val-Ala-Gly-Leu-Thr-Trp-Glu-Asp-Ser-Glu-Gly-Thr-Glu-Gly-Ser-

-Ser-Leu-Leu-Pro-Gly-Glu-Gln-Pro-Leu-His-Thr-Val-Asp-Pro-

Amino acid sequence of subunit having 214 amino acids

EP 0 276 551 B1

Glu-Glu-Val-Ser-Glu-Tyr-Cys-Ser-His-Met-Ile-Gly-Ser-Gly-His-Leu-Gln-Ser-Leu-Gln-

Arg-Leu-Ile-Asp-Ser-Gln-Met-Glu-Thr-Ser-Cys-Gln-Ile-Thr-Phe-Glu-Phe-Val-Asp-Gln-

Glu-Gln-Leu-Lys-Asp-Pro-Val-Cys-Tyr-Leu-Lys-Lys-Ala-Phe-Leu-Leu-Val-Gln-Asp-Ile-

Met-Glu-Asp-Thr-Met-Arg-Phe-Arg-Asp-Asn-Thr-Pro-Asn-Ala-Ile-Ala-Ile-Val-Gln-Leu-

Gln-Glu-Leu-Ser-Leu-Arg-Leu-Lys-Ser-Cys-Phe-Thr-Lys-Asp-Tyr-Glu-Glu-His-Asp-Lys-

Ala-Cys-Val-Arg-Thr-Phe-Tyr-Glu-Thr-Pro-Leu-Gln-Leu-Leu-Glu-Lys-Val-Lys-Asn-Val-

Phe-Asn-Glu-Thr-Lys-Asn-Leu-Leu-Asp-Lys-Asp-Trp-Asn-Ile-Phe-Ser-Lys-Asn-Cys-Asn-

Asn-Ser-Phe-Ala-Glu-Cys-Ser-Ser-Gln-Asp-Val-Val-Thr-Lys-Pro-Asp-Cys-Asn-Cys-Leu-

Tyr-Pro-Lys-Ala-Ile-Pro-Ser-Ser-Asp-Pro-Ala-Ser-Val-Ser-Pro-His-Gln-Pro-Leu-Ala-

Pro-Ser-Met-Ala-Pro-Val-Ala-Gly-Leu-Thr-Trp-Glu-Asp-Ser-Glu-Gly-Thr-Glu-Gly-Ser-

Ser-Leu-Leu-Pro-Gly-Glu-Gln-Pro-Leu-His-Thr-Val-Asp-Pro-Gly-Ser-Ala-Lys-Gln-Arg-

Pro-Pro-Arg-Ser-Thr-Cys-Gln-Ser-Phe-Glu-Pro-Pro-Glu-Thr-Pro-Val-Val-Lys-

Amino acid sequence of subunit having 238 amino acids

(c) Isoelectric point:

Isoelectric point (p I) measured by a polyacrylamide gel isoelectric focussing method and a sucrose density-gradient isoelectric focussing method is 3.1 - 3.7.

(d) Constitutive monosaccharides of sugar chain:

7

Analysis by a high-performance liquid chromatography after hydrolysis has identified mannose, galactose, N-acetylglucosamine, N-acetylgalactosamine and N-acetyl-neutraminic acid as constitutive monosaccharides of the sugar chain.

(e) Circular dichroism spectrum:

Far ultraviolet CD spectrum according to a circular dichroism dispersion meter has minimum peaks at 208nm and 222nm and reveals α-helix structure.

(f) Thermal stability:

Biological activity of the glycoprotein is not lost even by heating at 60 ± 0.5°C for 60 minutes.

(g) Infrared spectrum:

Infrared spectrum is as shown in Fig. 3.

WO-A-87/06954 discloses the preparation by recombinant technology, of a glycoprotein which appears to have 189, 223 or 224 amino acids of the same sequence as that of the first 223 or 224 amino acids of the above 238 amino acid protein. This document is acknowledged under Art 54(3)EPC.

The present invention provides the following methods for preparation of colony-stimulating factor, and also provides a glycoprotein having subunits each composed of the above 214 amino acid sequence.

Thus, there is provided a process, which comprises collecting a fraction of protein having a molecular weight of 70,000 daltons or more from normal human urine, subjecting the fraction to adsorption treatment by contacting with a hydrophobic affinity material equilibrated with 1 - 4 M salt-containing buffer and to elution treatment with a 0.5 - 1.0 M salt-containing buffer, subjecting the eluate to high-performance liquid gel filtration to recover a fraction containing a component of molecular weight of 70,000 - 150,000 daltons, and subjecting the fraction to reverse phase high-performance liquid chromatography at pH 1-2 to recover the colony-stimulating glyco-protein by a linear gradient concentration method with a solvent containing 0.1% trifluoroacetic acid; the above purification steps being carried out at a pH of 6.5 - 7.5 where not otherwise mentioned below. (This process is referred to as "the first preparation method" hereinafter).

The collection of the fraction having a molecular weight of 70,000 daltons or more is carried out in any conventional way, for example, by desalting and concentrating by ultrafiltration after clarification and contacting the urine with an anion exchanger and eluting with 0.2 - 0.4 M buffer, subjecting the eluate to gel filtration in a 1 - 4 M buffer, these steps being carried out at a pH of 6.5 - 7.5.

Further, there is provided a method for preparation of colony-stimulating glycoprotein factor which comprises isolating and purifying specific antibody against the present colony-stimulating glycoprotein from serum of mammals immunized with the colony-stimulating glycoprotein having stimulating action for formation of colony of monocyte-macrophage cells of mammals, conjugating this specific antibody to an insoluble support to prepare an antibody conjugated support, contacting a solution containing said colony-stimulating glycoprotein with said antibody conjugated support to adsorb the colony-stimulating glycoprotein contained in said solution and eluting the colony-stimulating glycoprotein from said antibody conjugated support. (This method is referred to as "the second preparation method" hereinafter.).

In more detail, the CSF of this invention may be prepared by the first and second methods as follows:

(1) Preparation of CSF (the first preparation method):

Normal human urine is adjusted to pH 8.0 - 9.0 to precipitate and remove viscous substances in the urine and the supernatant is concentrated and desalted by ultrafiltration which allows a cut-off at a molecular weight of 10,000 - 50,000 daltons.

The desalted human urine is concentrated to at least 200 times [at least 1% (w/v) in protein concentration], followed by adjusting pH to 6.5 - 7.5 and heat treating at 60°C for 10 hours if desired, for inactivation of virus, etc. The resulting precipitate is centrifugally removed and active ingredient is adsorbed onto an anion exchanger such as DEAE-cellulose, etc.

Then, this anion exchanger is washed with a 0.05 - 0.1 M buffer (pH 6.5 - 7.5) and then the active ingredient is eluted with a 0.2 - 0.4 M buffer (pH 6.5 - 7.5). This eluate is concentrated by ultrafiltration, if necessary, and is subjected to gel filtration by a gel filtrating agent, e.g., Sephacryl® S-300 (manufactured by Pharmacia Co.), which has been equilibrated with a buffer (pH 6.5 - 7.5) containing 1 M - 4 M salt such as ammonium sulfate or sodium chloride, to recover a fraction of 70,000 - 150,000 dalton in molecular weight.

Then, this fraction is subjected to adsorption treatment by contacting with a hydrophobic affinity material such as Phenyl-Sepharose (manufactured by Pharmacia Co.), which has been equilibrated with said 1 M-4 M salt containing buffer, and to elution treatment with a 0.5 - 1.0 M salt containing buffer (pH 6.5 - 7.5). The eluate is concentrated by ultrafiltration and subjected to gel filtration by a high-performance liquid gel filtration column, e.g., TSKG-3000SW (manufactured by Toyo Soda Inc.) to recover a fraction of a range of molecular weight of 70,000 - 150,000 daltons. This fraction is again concentrated and is subjected to adsorption treatment by a high-performance liquid reverse phase column e.g., Hi-Pore® RP-304 (manufactured by Biorad Co.), equilibrated with a 0.1% trifluoroacetic acid (TFA) solution (pH 1.2), and to elution treatment by linear gradient concentration method with a solvent e.g., acetonitrile or isopropanol containing 0.1% TFA. The thus obtained CSF is a pure substance having a specific activity of at least 1 x $10^8$ units/mg•protein.

(2) Physico-chemical properties of CSF:

The CSF of this invention prepared as mentioned above has the following physico-chemical properties:

(a) Molecular weight:

Molecular weight of the CSF was measured by sodium dodecyl sulfate polyacrylamide gel electrophoresis in the absence of reducing agent in accordance with the method of Laemmli (Nature, vol. 227, pages 680-685, 1970) to obtain 70,000 - 90,000 daltons.

Then, the CSF was reduced with 0.2 M mercapto ethanol to cause dissociation to subunits and the molecular weight of each subunit was measured in the same manner and found to be 35,000 - 45,000 dalton. (Fig. 1).

Fig. 1 is an electrophoresis diagram of sodium dodecyl sulfate polyacrylamide gel electrophoresis of the CSF of this invention wherein A-E indicate non-reduced CSF (dimers), F and G indicate molecular weight marker proteins and H-L indicate reduced CSF (subunit) and ordinates indicate molecular weight (x $10^3$ dalton).

(b) Amino acid sequence of subunit protein:

The $NH_2$-terminal amino acid sequence was determined by analysis of purified CSF by the usual method using a gas phase amino acid sequencer. Thus, the purified CSF was modified with 6 M guanidine, alkylated with monoiodoacetic acid, then desalted and subjected to trypsin digestion and cyanogen bromide cleavage. The trypsin digested and cyanogen bromide cleaved peptides were fractionated by Vydac C-18 reverse phase high-performance liquid chromatography to obtain cleaved peptide fractions and each fraction was analyzed by the gas phase amino acid sequencer to analyze the amino acid sequence of the peptide fragment. Conforming to the sequence predicted by human CSF-1 encoding mRNA, the primary structure of subunit protein was determined by alignments of trypsin digested and cyanogen bromide cleaved peptide sequences. The results revealed that the subunit contains an amino acid sequence of 189, 214 or 238 amino acids represented by the following formulae.

The amino acids from glutamic acid, which is the $NH_2$-terminal amino acid to the 149th glutamine are the same as those of a known CSF-1, but the 40, 65 or 89 amino acids from the 150th to the 189th, 214th or 238th respectively are utterly different from those of a known CSF-1.

EP 0 276 551 B1

1
Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-
50
-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-
100
-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-
122                                                                              140
-Phe-Asn-Glu-Thr-Lys-Asn-Leu-Leu-Asp-Lys-Asp-Trp-Asn-Ile-Phe-Ser-Lys-Asn-Cys-Asn-
150
-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-
189
-Pro-Ser-Met-Ala-Pro-Val-Ala-Gly-Leu-   α-β-γ


Amino acid sequence of subunit having 189 amino acids

EP 0 276 551 B1

Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

-Phe-Asn-Glu-Thr-Lys-Asn-Leu-Leu-Asp-Lys-Asp-Trp-Asn-Ile-Phe-Ser-Lys-Asn-Cys-Asn-

-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

-Pro-Ser-Met-Ala-Pro-Val-Ala-Gly-Leu-Thr-Trp-Glu-Asp-Ser-Glu-Gly-Thr-Glu-Gly-Ser-

-Ser-Leu-Leu-Pro-Gly-Glu-Gln-Pro-Leu-His-Thr-Val-Asp-Pro-   , and


Amino acid sequence of subunit having 214 amino acids

Glu-Glu-Val-Ser-Glu-Tyr-Cys-Ser-His-Met-Ile-Gly-Ser-Gly-His-Leu-Gln-Ser-Leu-Gln-
-Arg-Leu-Ile-Asp-Ser-Gln-Met-Glu-Thr-Ser-Cys-Gln-Ile-Thr-Phe-Glu-Phe-Val-Asp-Gln-
-Glu-Gln-Leu-Lys-Asp-Pro-Val-Cys-Tyr-Leu-Lys-Lys-Ala-Phe-Leu-Leu-Val-Gln-Asp-Ile-
-Met-Glu-Asp-Thr-Met-Arg-Phe-Arg-Asp-Asn-Thr-Pro-Asn-Ala-Ile-Ala-Ile-Val-Gln-Leu-
-Gln-Glu-Leu-Ser-Leu-Arg-Leu-Lys-Ser-Cys-Phe-Thr-Lys-Asp-Tyr-Glu-Glu-His-Asp-Lys-
-Ala-Cys-Val-Arg-Thr-Phe-Tyr-Glu-Thr-Pro-Leu-Gln-Leu-Leu-Glu-Lys-Val-Lys-Asn-Val-
-Phe-Asn-Glu-Thr-Lys-Asn-Leu-Leu-Asp-Lys-Asp-Trp-Asn-Ile-Phe-Ser-Lys-Asn-Cys-Asn-
-Asn-Ser-Phe-Ala-Glu-Cys-Ser-Ser-Gln-Asp-Val-Val-Thr-Lys-Pro-Asp-Cys-Asn-Cys-Leu-
-Tyr-Pro-Lys-Ala-Ile-Pro-Ser-Ser-Asp-Pro-Ala-Ser-Val-Ser-Pro-His-Gln-Pro-Leu-Ala-
-Pro-Ser-Met-Ala-Pro-Val-Ala-Gly-Leu-Thr-Trp-Glu-Asp-Ser-Glu-Gly-Thr-Glu-Gly-Ser-
-Ser-Leu-Leu-Pro-Gly-Glu-Gln-Pro-Leu-His-Thr-Val-Asp-Pro-Gly-Ser-Ala-Lys-Gln-Arg-
-Pro-Pro-Arg-Ser-Thr-Cys-Gln-Ser-Phe-Glu-Thr-Pro-Pro-Glu-Thr-Pro-Val-Val-Lys-

Amino acid sequence of subunit having 238 amino acids

In the subunit containing the amino acid sequence of 189 amino acids, it is presumed that further amino acids, α, β and γ, may exist subsequently to the 189th leucine and that α, β and γ are threonine, tryptophane and glutamic acid respectively.

For the COO-terminal amino acid, there were detected leucine at the 189th, proline at the 214th and lysine at the 238th depending on molecular weight of the subunit protein. Asparagines at the 122nd and 140th have typical N-glycosidic linkage of Asn-X-Ser/Thr and it was presumed that a sugar chain was linked at this position. The "X" indicates an optional amino acid.

EP 0 276 551 B1

(c) Constitutive monosaccharides of sugar chain

Constitutive monosaccharides of sugar chains binding to the polypeptide were freed by hydrolysis and then analyzed by high-performance liquid chromatography. Aldose and sialic acid were fractionated by an anion exchange column and hexosamine was fractionated by a cation exchange column according to the boric acid buffer concentrated gradient elution method and were post-column labelled with cyanoacetamide or arginine and then identified by the fluorescence method. The sugar chain contained in this CSF was not uniform and quantitative analysis was difficult, but mannose, galactose, N-acetylglucosamine, N-acetylgalactosamine and N-acetylneuraminic acid were identified as constitutive monosaccharides.

(d) Isoelectric point

Isoelectric point was measured by the polyacrylamide gel isoelectric focussing method and the sucrose density-gradient isoelectric focussing method to obtain pI of 3.1 - 3.7.

(e) Circular dichroism (CD) spectrum

CD spectrum in the far ultraviolet region was measured by a dichrograph (J-600 manufactured by JASCO CO.) (Fig. 2).
Fig. 2 shows the CD spectrum of CSF of this inventon wherein the abscissa axis indicates wavelength (nm) and the ordinate axis indicates ellipticity (mdeg). Minimum peaks were recognized at wavelengths of 208 nm and 222 nm and it was presumed that the secondary structure of the CSF contained an $\alpha$-helix structure.

(f) Thermal stability

The CSF was dissolved at a concentration of 1 $\mu$g/ml in a dilute buffer (pH 7.0) and heated at 60 $\pm$0.5°C for 60 minutes and colony-stimulating activity (referred to hereafter) was measured to find substantially no reduction in the activity.

(g) Infrared absorption spectrum

The infrared absorption spectrum of lyophilized powder of this CSF measured by the transmission method (KBr tablet) using a Fourier transform infrared spectrometer (5DXC manufactured by Nicolet Co.) is as shown in Fig. 3. The abscissa axis in Fig. 3 indicates wave-number ($cm^{-1}$) and the ordinate axis indicates percent transmission.
This CSF showed intense absorption at 1650 $cm^{-1}$, 1201 $cm^{-1}$ and 1133 $cm^{-1}$ and medium absorption at 1537 $cm^{-1}$, 1432 $cm^{-1}$ and 1068 $cm^{-1}$.

(3) Preparation of CSF (the second preparation method)

The second preparation method comprises the following three steps:

① Preparation of specific antibody against colony-stimulating glycoprotein (referred to as "anti-CSF antibody" hereinafter):

Mammals such as rabbits, goats, sheep and horses are immunized with CSF of this invention obtained by the above first preparation method or the separately effected second preparation method. That is, CSF of this invention is dissolved in a physiological saline at a concentration of 0.1 - 1.0 mg/ml and is mixed with equal amount of a complete Freund's adjuvant. The mixture is subcutaneously administered to mammals for 4 - 8 weeks once or twice a week to immunize them. When blood antibody titers of the immunized animals are increased, additional immunization is carried out by intravenous or subcutaneous injection and blood is collected on third-7th days after the additional immunization and CSF antiserum is collected. Antibody titer of the collected antiserum against CSF is measured by a CSF biological titer neutralization test mentioned hereinafter and for anti-CSF antibody titer in antiserum, it is preferred to select an antiserum which neutralizes CSF biological activity of at least 5 x $10^6$ units per 1 ml. The collected antiserum is purified by ammonium sulfate salting-out twice and DEAE-cellulose chromatography to prepare anti-CSF antibody of immunoglobulin G or M fraction. If necessary, the anti-CSF antibody is further purified by

13

passage through an antigen column having, as a ligand, impurity protein which shows cross reaction with CSF or anti-CSF antibody to absorb only the anti-CSF antibody or absorb the impurity protein.

② Preparation of antibody conjugated support:

As insoluble support capable of bonding CSF antibody, there may be used any of the known supports which can chemically bind to an $NH_2$-group or COOH-group of the antibody protein. These include, for example, cyanogen bromide activated or epoxidized polysaccharide gel, formylated polysaccharide gel, or aminoethylated or hydrazidated polymer. The binding reaction between the insoluble support and the anti-CSF antibody differs in its reaction conditions depending on the binding group of the insoluble supports selected and hence an antibody is chosen to achieve optimum conditions. For example, antibody is dissolved in a carbonate buffer of pH 8 - 10 in the case of a cyanogen bromide activated support, in a solution of pH 10 or more in the case of an epoxidized support and in a neutral solution in the case of a formylated support. The temperature condition of the binding reaction also varies depending on the insoluble support, but in the case of the binding reaction of this invention is is preferred to carry out the reaction at a low temperature of 25°C or lower. Especially, in the case of cyanogen bromide activated support, the reaction is effected at 4°C or lower. The amount of antibody to be bonded is 10 - 50 mg, preferably 20 - 30 mg per 1 g (wet weight) of the insoluble support and the antibody concentration at binding reaction is adjusted to 1 - 4% (w/v). After completion of the binding reaction, reactive groups remaining in the support unbound to antibody are inactivated by suitable treatments to obtain an antibody conjugated support.

③ Purification of CSF by the antibody conjugated support:

The antibody conjugated support is washed with a buffer of pH 6 - 8 which contains 0.5 - 1.0 M salt, e.g., sodium chloride. The washed antibody conjugated support is packed in a column of suspended in a buffer. The former is used as a column chromatography and the latter is used as a batch-wise chromatography. As a solution containing CSF of this invention, there may be used, for example, a human urine concentrate, a CSF forming cell culture solution supernatant or a CSF gene recombined cell culture solution supernatant. This is adjusted to pH 6 -8 and the equilibrated with the same buffer as used for washing of the antibody conjugated support above or sodium chloride is added thereto at a concentration of 0.5 - 1.0 M. The thus treated solution is allowed to come into contact with the antibody conjugated support. This contacting is carried out in the column or batch-wise chromatography. In the case of the column type chromatography, the solution is passed therethrough at lower than room temperature, preferably 10°C or lower and at a flow rate of 5 - 20 ml/cm²•hr to adsorb CSF to the column of antibody conjugated support. The amount of CSF used is desirably 5 million -20 million units per 1 g (wet weight) of antibody conjugated support. After completion of adsorption, the buffer is passed through to remove contaminants. In the case of batch-wise chromatography, the solution is mixed with antibody conjugated support at room temperature or 10°C or lower and the mixture is stirred for 1 - 10 hours and then is filtered by, for example, a glass filter, to recover the antibody conjugated support. This antibody conjugated support is washed with the buffer to completely remove contaminants. CSF which has been specifically adsorbed onto the antibody conjugated support is eluted with a dissociation solution of an antigen-antibody complex, for example, acetate buffer of pH 2 - 3, 3 - 4 M of thiocyanate, or 0.1 - 0.2 M of 2,4-dinitrophenol, from the antibody conjugated support. CSF is eluted by passing an eluting solution through the column in the case of the column chromatography and by suspending the antibody conjugated support in an eluting solution and stirring the suspension in the case of the batch-wise chromatography. The thus obtained CSF is a pure CSF containing no impurities. The second preparation method of this invention comprises the above steps ① - ③ . This will be explained in more detail by the following experimental examples.

Experimental Example 1: Preparation of anti-CSF antibody using rabbits.

The CSF of this invention prepared by the first preparation method was dissolved in a physiological saline at a concentration of 0.4 mg/ml and this solution was mixed with an equal amount of a complete Freund's adjuvant. A 0.5 ml of this mixture was subcutaneously administered to the back of rabbits (white) of body weight 2.5 - 3.5 kg once a week for 4 weeks to immunize them and the rabbits were subjected to a booster by intravenous injection in the 5th week. Blood was collected on the 5th day after the booster to obtain anti-CSE serum. During the immunization, blood was collected from an ear vein once a week to test the increase in CSF antibody titer.

Anti-CSF antibody titer was obtained by the following CSF biological activity neutralization test. That is, the collection serum was diluted stepwise with physiological saline and 0.5 ml thereof was mixed with 0.5 ml of CSF solution (2,000 units/ml, 0,02 $\mu$g/ml) at room temperature for 5 minutes. Then, 0.1 ml of the mixture was added to 1 ml of McCoy's 5A medium containing $1 \times 10^5$ mouse bone marrow cells, 20% of fetal calf serum and 0.3% agar and this was incubated in a humidified 7.5% $CO_2$ atmosphere at 37°C for 7 days. Then, colonies formed by CSF were counted. Anti-CSF antibody titer was obtained by the following formula:

Anti-CSF antibody titer (neutralizing unit/ml) = (the number of colonies formed without addition of serum) - (the number of colonies formed with addition of serum) x (dilution rate of serum (time)) x 10

Change of blood anti-CSF antibody titer of three rabbits immunized with CSF is shown in Table 1.

Table 1

| Anti-CSF antibody titer in serum of rabbits | | | |
|---|---|---|---|
| Immunization week | Antibody titer (x $10^4$ neutralization unit/ml) | | |
| | Rabbit No. 1 | Rabbit No. 2 | Rabbit No. 3 |
| 0 (before immunization) | <0.01 | <0.01 | <0.01 |
| After one week | <0.01 | <0.01 | <0.01 |
| After 2 weeks | 96 | 24 | 66 |
| After 3 weeks | 384 | 88 | 196 |
| After 4 weeks | 512 | 92 | 482 |
| 5 weeks (Booster) | 1,200 | 380 | 1,100 |

Each of 50 ml portions of the anti-serum of rabbits no. 1 and No. 3 in the above Table 1 was purified by the following operation. The purification was carried out at 4°C. To 50 ml of the antiserum was added under stirring a saturated ammonium sulfate solution at a concentration of 40% (v/v) to precipitate antibody. The precipitate was redissolved in a distilled water and thereto was again added a saturated ammonium sulfate solution at a concentration of 33% (v/v) to precipitate antibody. The precipitate was dissolved in a distilled water and the solution was subjected to dialysis against a 0.02 M tris-HCl buffer (pH 8.6) and then passed through DEAE-cellulose column previously equilibrated with said tris-HCl buffer to collect unadsorbed IgG fractions, which were purified as anti-CSF antibody of IgG fractions. The obtained anti-CSF antibodies were 480 mg and 460 mg, respectively.

Experimental Example 2 Preparation of antibody conjugated support

Optimum binding conditions of insoluble supports with anti-CSF antibody were examined using cyanogen bromide activated Sepharose 4B (manufactured by Pharmacia Co.) and Formyl-Cellulofine (manufactured by Chisso Co.) as the insoluble supports. When cyanogen bromide activated Sepharose 4B was used, anti-CSF antibody was dissolved in 0.1 M carbonate buffer (pH 9.0) to obtain an antibody solution. Cyanogen bromide activated Sepharose 4B was swollen in 1 mM hydrochlorid acid solution and washed with 0.1 M carbonate buffer. Then, the buffer was removed by suction filtration and 1 g (wet weight) of cyanogen bromide activated Sepharose 4B gel was placed in a 50 ml conical flask. Thereto was added a 2 ml portion of each of antibody solutions of four concentrations as shown in Table 2 and each of them was shaken at 4°C for 18 hours to allow binding of the antibody to the support. After completion of the binding the support was washed with 0.1 M carbonate buffer and then reactive groups of the support were inactivated with 0.2 M tris-HCl buffer (pH 7.0).

When Formyl-Cellulofine was used, anti-CSF antibody was dissolved in 0.1 M sodium phosphate buffer (pH 7.0) and this was used as an antibody solution. Formyl-Cellulofine was washed with distilled water and 0.1 M sodium phosphate and then the buffer was removed by suction filtration and 1 g (wet weight) of Formyl-Cellulofine was placed in a 50 ml conical flask. Thereto was added a 2 ml portion of each of the antibody solutions of four concentrations as shown in Table 3. Each of them was stirred at room temperature for 2 hours, followed by adding 7 mg of sodium cyanoborohydride and further stirring at room temperature for 10 hours to bond the antibody to the support. After binding, the support was washed with 0.2 M tris-HCl buffer (pH 7.0) and thereto was added 2 ml of tris-HCl buffer containing 5 mg of sodium cyanoborohydride, followed by stirring at room temperature for 4 hours to inactivate reactive groups.

Amounts of antibody which bonded to cyanogen bromide activated Sepharose 4B and Formyl-Cellulofine are shown in Tables 2 and 3.

Table 2

| Amount of antibody bounded to cyanogen bromide activated Sepharose 4B | | | |
|---|---|---|---|
| Concentration of antibody (mg/ml) | Amount of antibody solution added (ml) | Binding amount to support (mg/g gel) | Binding rate (%) |
| 10 | 2 | 16.4 | 82.0 |
| 20 | 2 | 28.0 | 70.0 |
| 30 | 2 | 30.5 | 50.8 |
| 40 | 2 | 36.8 | 46.0 |

Table 3

| Amount of antibody bounded to Formyl-Cellulofine | | | |
|---|---|---|---|
| Concentration of antibody (mg/ml) | Amount of antibody solution added (ml) | Binding amount to support (mg/g gel) | Binding rate (%) |
| 13.9 | 2 | 22.4 | 80.6 |
| 20.3 | 2 | 34.0 | 83.7 |
| 30.1 | 2 | 42.1 | 69.9 |
| 40.0 | 2 | 48.3 | 60.4 |

Antibody concentration for bonding anti-CSF antibody efficiently and in a large amount to the insoluble support was 20 - 30 mg/ml in both cyanogen bromide activated Sepharose 4B and Formyl-Cellulofine. In the case of the lower concentrations, the binding rate was high, but the amount of antibody bound was small and in the case of the higher concentrations, the amount bound was large, but the binding rate was low.

Experimental Example 3 Preparation of CSF by antibody support

An antibody conjugated support comprising Formyl-Cellulofine as insoluble support and having an antibody binding amount of 31 mg/g gel was prepared in the same manner as in Experimental Example 2. A solution containing a partially purified CSF of specific activity $2 \times 10^5$ unit/mg (purity: less than 1%) which was obtained by subjecting human urine to DEAE-cellulose treatment and gel filtration was used as CSF and examination on purification of CSF was effected by batch-wise chromatography.

The antibody conjugated support was washed with 0.02 M phosphate buffer (pH 7.0) containing 0.5 M sodium chloride and subjected to suction filtration.

This CSF containing solution was previously equilibrated with said phosphate buffer. To 1 g of the antibody conjugated support was added the CSF containing solution in the 4 respective addition amounts as shown in Table 4, followed by stirring at 10°C for 6 hours and then washing sufficiently with said buffer and CSF was eluted with 0.2 M acetate-hydrochloric acid buffer (pH 2.5).

Table 4 Preparation of CSF by antibody conjugated support using Formyl-Cellulofine as insoluble support

| Sample No. | Amount of CSF charge | | Amount of CSF recovered | |
|---|---|---|---|---|
| | Addition amount (ml) | Total amount (unit) | total activity (unit) | Recovery (%) |
| 1 | 2 | $0.6 \times 10^7$ | $0.58 \times 10^7$ | 96.7 |
| 2 | 5 | $1.5 \times 10^7$ | $1.03 \times 10^7$ | 68.7 |
| 3 | 10 | $3.0 \times 10^7$ | $1.21 \times 10^7$ | 40.3 |
| 4 | 20 | $6.0 \times 10^7$ | $2.06 \times 10^7$ | 34.3 |

As shown in Fig. 4, in order to purify CSF in high recovery, it was suitable to charge CSF in an amount of 0.6 - 1.5 x 10$^7$ units per 1 g (wet weight) of antibody conjugated support. Further, the recovered CSF was subjected to the tests for specific activity and purity by SDS-PAGE.

The results are shown in Table 5.

Table 5

| Specific activity and purity of CSF | | | |
|---|---|---|---|
| Sample No. | Specific activity ($\times 10^7$ unit/mg) | Purification fold | Purity (%) |
| 1 | 7.5 | 375 | >95 |
| 2 | 7.4 | 370 | >95 |
| 3 | 5.8 | 290 | >90 |
| 4 | 4.9 | 245 | >90 |

As shown in Table 5, the partially purified CSF was purified at least 245 fold by affinity-chromatography with Formyl-Cellulofine and the product was a homogeneous glycoprotein having a purity of more than 90% according to SDS-PAGE.

A virus present in the CSF of the present invention, if any, can be inactivated by heating at 60°C for 10 hours, at any stage of the first and second preparation methods, due to the thermal stability of the present CSF.

BIOLOGICAL ACTIVITY OF CSF

(a) Colony-stimulating activity and specific activity:

Colony-stimulating activity of the CSF of this invention was determined by a colony formation method by using monolayer soft agar gel employing mouse bone marrow cell. Thus, each CSF sample was mixed with 1 ml of McCoy's 5A medium containing 0.3% of agar, 20% of fetal calf serum (FCS) and $1 \times 10^5$ mouse bone marrow cells and the mixture was incubated for 7 days at 37°C in a humidified carbon dioxide atmosphere. After incubation, the number of colonies formed was counted, the term "colony" herein meaning a cell aggregate containing at least 50 cells. The colony stimulating activity was expressed in units. One unit means the amount of CSF required for forming one colony. The specific activity was expressed by the number of colonies (units) formed per 1 mg of CSF protein. It was found that the CSF of this invention had a specific activity of $1.4 \times 10^8$ unit/mg protein. The formed colonies were subjected to hematoxylin-eosin staining and morphologically classified to find that at least 95% of the colonies were monocytes-macrophages.

(b) Stimulating effects of proliferation of mouse bone marrow monocyte-macrophage stem cells (CFU-M) in vitro and in vivo.

(b)-1: In vitro test

Non adherent $C_{57}BL$ mouse bone marrow cells prepared by using a plate adherent method were suspended in McCoy's 5A medium containing 20% FCS at a concentration of $1 \times 10^6$ cells/ml and added with 0 (control), 100 units/ml, 500 units/ml, 1,000 units/ml and 2,000 units/ml of the CSF purified by the first preparation method of this invention, and these media were incubated at 37°C for 24 hours in a humidified 7.5% $CO_2$ atmosphere. After incubation, the bone marrow cells were centrifugally washed and diluted 5 times with the same medium without the CSF. A 0.1 ml aliquot of the diluted bone marrow cell solution of each sample was dispensed into 4 culture dishes and mixed with 1 ml of McCoy's 5A medium supplemented with 1,000 units of CSF, 0.3% agar and 20% FCS. They were incubated at 37°C for 7 days in a humidified 7.5% $CO_2$ atmosphere. After incubation, the number of CFU-M formed was counted. In this case, a cell aggregate containing at least 50 cells was judged as monocyte-macrophage stem cell (CFU-M). The results are shown in Table 6.

Table 6

| CFU-M proliferation stimulating action in vitro | |
| --- | --- |
| Dosage of CSF (unit/ml) | The number of CFU-M ($\times 10^3/10^6$ Bone marrow cell) |
| 0 | 3.5 ± 0.1 |
| 100 | 4.6 ± 0.2 |
| 500 | 5.1 ± 0.4* |
| 1,000 | 7.4 ± 0.3** |
| 2,000 | 7.8 ± 0.3** |

Note: * and ** indicate level of significance of 5% and 1%, respectively.

As shown in Table 6, the number of CFU-M in mouse bone marrow cells increased depending on concentration of CSF added.

(b)-2: In vivo test

To $C_{57}BL$ mice (5/group) were administered by intraperitoneal injection CSF in an amount of 0 (physiological saline), 80 x $10^4$ units/kg body weight, 160 x $10^4$ units/kg and 320 x $10^4$ units/kg, once a day, for 3 consecutive days. On the next day after completion of administration, bone marrow of femur and spleen were removed from each mouse and the number of monocyte-macrophage stem cells (CFU-M) per organ was measured by the same procedures mentioned in the above in vitro test. The results are as shown in Tables 7 and 8.

Table 7

| Stimulating action on proliferation of mouse bone marrow of femur CFU-M | |
| --- | --- |
| Dosage of CSF ($\times 10^4$ unit/kg) | The number of CFU-M ($\times 10^4$ Bone marrow of femur) |
| 0 | 3.05 ± 0.65 |
| 80 | 3.82 ± 0.66 |
| 160 | 5.23 ± 0.36** |
| 320 | 5.81 ± 0.57** |

Note: ** indicates level of significance of 1%.

Table 8

| Stimulating action on proliferation of mouse spleen CFU-M | |
| --- | --- |
| Dosage of CSF ($\times 10^4$ units/kg) | The number of CFU-M ($\times 10^4$ units/spleen) |
| 0 | 1.09 ± 0.42 |
| 80 | 1.25 ± 0.49 |
| 160 | 2.20 ± 0.38** |
| 320 | 1.91 ± 0.74** |

Note: ** indicates level of significance of 1%.

As shown in Tables 7 and 8, administration of 80 x $10^4$ units/kg body weight of CSF resulted in increase of CFU-M in both bone marrow and spleen, and administration of 160 x $10^4$ units/kg resulted in a remarkable increase.

The CSF of this invention which has the above stated physicochemical properties and biological activity is compared with a known similar substance as follows.

CSF of this invention is a glycoprotein having the structure of a biologically active homodimer wherein two polypeptide subunits containing sugar chains are, like the known CSF-1, bonded through disulfide bond,

19

the glycoprotein having a molecular weight of 70,000 - 90,000 daltons which is more than that of CSF-1. Furthermore, a glycopolypeptide subunit of the CSF of this invention has at least 189, 214 or 238 amino acids and has a molecular weight of 35,000 - 45,000 dalton which is more than the (roughly) 20,000 - 25,000 daltons of the glycopolypeptide subunit of the known CSF-1; see Das et al, J. Biol. Chem., (1982), 252(22), 13679-13681, and Shieh et al, Archives of Biochemistry, (15 Feb 1987), 253(1), 205-213. The amino acid sequence of the subunit is compared with that of the known CSF-1 to find that the amino acid sequence from the first to the 149th at the $NH_2$-terminus is the same, but that from the 150th to the 189th, 214th or 238th is different from that estimated for the cDNA of CSF-1 and is not coded by the CSF-1 gene. Thus, it has been found that the CSF of this invention is partially common to CSF-1, but is different from the known CSF-1 both genetically and structurally. Further, the CSF of this invention is completely different from the known HGI-glycoprotein and CSF-HU in its biological and physicochemical properties.

Examples of this invention are shown below.

Example 1

200 liters of normal human urine was adjusted to pH 8.5 and the precipitate was removed by filtration. The filtrate was concentrated and desalted by an ultrafiltration membrane (membrane is H10x50 manufactured by Amicon Corp.) through which fractions having molecular weight of 50,000 daltons or more cannot pass. Then, the concentrated liquid was adjusted to pH 7.0 and heat-sterilized at 60° for 10 hours in a closed vessel. After sterilization, the concentrated liquid was subjected to centrifugation (5,000 x g, 30 minutes) to remove precipitate and then was mixed with DEAE-cellulose equilibrated with 0.02 M phosphate buffer (pH 7.2) to adsorb the liquid. This DEAE-cellulose was washed with 0.02 M phosphate buffer and 0.02 M phosphate buffer (pH 7.2) containing 0.05 M sodium chloride and then eluted with 0.02 M phosphate buffer (pH 7.2) containing 0.25 M sodium chloride. The eluate was concentrated by ultrafiltration (membrane is H1P10 manufactured by Amicon Corp.) and gel-filtered with 1 M ammonium sulfate added buffer (pH 7.2) using Sephacryl S-300 (∅4 x 80 cm, manufactured by Pharmacia Co.). A fraction of 70,000 - 150,000 daltons in molecular weight obtained by the gel -filtration was adsorbed onto Phenyl-Sepharose 4B column (∅2 x 20 cm, manufactured by Pharmacia Co.) equilibrated with said 1 M ammonium sulfate buffer and then eluted with 0.5 M ammonium sulfate added buffer (pH 7.2). The eluate was concentrated by ultrafiltration (membrane is NM-3 manufactured by Asahi Kasei Kogyo K.K.) and subjected to high-performance liquid chromatography by passing through TSKG-3,000 SW column (∅4 x 600 mm x 2, manufactured by Toyo Soda Inc.) to obtain a fraction of 70,000 - 150,000 daltons in molecular weight. This fraction was concentrated again and subjected to high-performance liquid chromatography by linear gradient concentration of acetonitrile 0 - 100% (pH 2.0) containing 0.1% trifluoroacetic acid through reverse phase column of Hi-Pore RP-304 (∅4 x 150 mm, manufactured by Biorad Co.) to elute CSF to obtain a purified CSF of $1.4 \times 10^8$ units/mg protein in specific activity. Degree of purification of CSF at each step is as shown in Table 9.

Table 9  Purification of CSF

| Step of purification (note) | Protein (mg) | Specific activity (unit/mg) | Purification rate (time) | Recovery (%) |
|---|---|---|---|---|
| (1) DEAE-cellulose | 733.6 | $1.6 \times 10^5$ | 1 | 100 |
| (2) Sephacryl S-300 | 149.4 | $5.7 \times 10^5$ | 3.6 | 72.6 |
| (3) Phenyl-Sepharose | 11.3 | $8.8 \times 10^6$ | 55.0 | 85.4 |
| (4) TSKG-3,000 SW | 2.5 | $2.0 \times 10^7$ | 125.0 | 43.6 |
| (5) Hipor-RP-304 | 0.25 | $1.4 \times 10^8$ | 875.0 | 29.9 |

(Note)  Since CSF inhibitor is present in untreated human urine and exact activity cannot be measured, activities after DEAE-cellulose treatment are stated.

Example 2

Example 1 was repeated except that Sephacryl S-300 was replaced by TSKG-3,000SW (HLC-837 manufactured by Toyo Soda Inc.) and Phenyl-Sepharose 4B was replaced by TSK Phenyl-5PW (manufactured by Toyo Soda Inc.) and purifications were all carried out by high-performance liquid chromatography. The obtained CSF has a specific activity of $1.5 \times 10^8$ units/mg and recovery was similar to that of Example 1.

Example 3

Anti-CSF antiserum was collected from ten rabbits immunized with CSF obtained in Example 1 and having sufficiently increased antibody titer, and about 4 g of anti-CSF antibody purified by the method of Experimental Example 1 was obtained. The anti-CSF antibody was dialyzed in 0.1 M phosphate buffer (pH 7.0) and was adjusted to 20 mg/ml concentration. 200 ml of this antibody solution was added to 100 g of Formyl-Cellulofine previously washed with distilled water and 0.1 M phosphate buffer, and the mixture was stirred at room temperature for 2 hours. Then, thereto was added 700 mg of sodium cyanoborohydride, followed by further stirring for 16 hours to bond the anti-CSF antibody to the Formyl-Cellulofine to obtain an antibody conjugated support. After binding, this support was washed with 0.2 M tris-HCl buffer, followed by adding thereto 200 ml of tris-HCl buffer containing 500 mg of sodium cyanoborohydride and stirring at room temperature for 4 hours to inactivate unreacted groups. Then, this antibody conjugated support was sufficiently washed with 0.02 M phosphate buffer containing 0.5 M NaCl. The antibody conjugated support had 29.5 mg/g support of anti-CSF antibody bonded thereto. Then, 1,000 liters of normal human urine was concentrated by ultrafiltration and desalted and then was adsorbed onto DEAE-cellulose to remove contaminates and eluted with 0.3 M NaCl solution. To the eluate was added sodium chloride at a concentration of 0.5 M to obtain a solution containing CSF. This CSF had a specific activity of $2 \times 10^5$ units/mg. To 100 g of said antibody conjugated support was added said solution containing CSF (total amount 500 ml), followed by stirring at 10°C or lower overnight and subjecting to batch-wise chromatography. Then, the mixture was filtered by glass filter to collect antibody conjugated support, which was sufficiently washed with 0.02 M phosphate buffer containing 0.5 M NaCl. Then, thereto was added 500 ml of 0.2 M acetate buffer (pH 2.5), followed by stirring at 10°C for 1 hour to elute CSF. The eluate was adjusted to pH 7.0 and then concentrated and desalted by ultrafiltration to obtain about 10 mg of purified CSF. This purified CSF had a specific activity of $5.2 \times 10^7$ units/mg and a purity of at least 90% according to the SDS-PAGE method.

Example 4

10 g of the same antibody conjugated support as prepared in Example 3 was packed in a column (⌀15 x 100 mm) and washed by passing therethrough 0.02 M phosphate buffer (pH 7.0) containing 1.0 M NaCl. Isolation and purification of CSF from CSF producing cell culture supernatant were carried out by using said column. Human pancreatic cancer cells (referred to as "MIA-PaCa-2 cells") were used as the CSF producing cells. MIA-PaCa-2 cells were incubated in MEM medium of Dulbecco containing 5% of fetal calf serum for 7 days, and 1 liter of this culture supernatant was concentrated by ultrafiltration to prepare a solution containing CSF. This CSF has a specific activity of $4 \times 10^5$ units/mg. To 100 ml of this solution was added sodium chloride at a concentration of 1.0 M and the solution was passed through said column at 10°C. Thereafter, the column was washed with a phosphate buffer containing 1.0 M NaCl and then CSF was eluted with 3.5 M sodium thiocyanate. The eluate was dialyzed in 0.02 M phosphate buffer and then concentrated by ultrafiltration to obtain about 1 mg of purified CSF. This CSF had a specific activity of $7.8 \times 10^7$ units/mg and a purity of at least 90% according to the SDS-PAGE method.

As explained above, this invention can provide novel CSF which stimulates colony formation of monocyte-macrophage cells of mammals.

According to the first preparation method of CSF of this invention, CSF can be purified from human urine which is easily available. According to the second preparation method, other solutions containing CSF such as culture solution of CSF producing cells or CSF gene recombination cells can be used, and also CSF of this invention can be selectively isolated and purified from solutions containing various contaminants and other factors by using antibody conjugated support. Because of its superior action for removal of different kinds of proteins other than human protein, highly purified CSF can be provided inexpensively as medicines.

A CSF in accordance with the invention comprises a homodimer having two glycopolypeptides each having the 214 amino acid sequence given above.

The asparagines at positions 122 and 140 may have respective N-glycosidic linkage sites provided by asparagine (Asn)-X-threonine (Thr) and asparagine (Asn)-X-serine (Ser) respectively, where X is an optional amino acid.

**Claims**

1.  A colony-stimulating glycoprotein having stimulating action on colony formation of monocyte-macrophage cells of mammals and having a molecular weight of 70,000 - 90,000 daltons as measured by the non-reducing sodium dodecyl sulfate polyacrylamide electrophoresis method, which glycoprotein is a homodimer of two glycopolypeptide subunits, which subunits (a) are composed of respective amino acid sequences which are the same as one another, (b) are bonded to one another through S-S linkage, and each of which subunits (c) has a molecular weight of 35,000 - 45,000 daltons as measured by the said non-reducing electrophoresis method, and (d) each consists of the 214 amino acid sequence represented by the formula:

Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

-Phe-Asn-Glu-Thr-Lys-Asn-Leu-Leu-Asp-Lys-Asp-Trp-Asn-Ile-Phe-Ser-Lys-Asn-Cys-Asn-

-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

-Pro-Ser-Met-Ala-Pro-Val-Ala-Gly-Leu-Thr-Trp-Glu-Asp-Ser-Glu-Gly-Thr-Glu-Gly-Ser-

-Ser-Leu-Leu-Pro-Gly-Glu-Gln-Pro-Leu-His-Thr-Val-Asp-Pro.

2. A colony-stimulating glycoprotein of Claim 1, wherein the 122nd and 140th asparagines in said amino acid sequences have N-glycosidic linkage sites represented by asparagine (Asn)-X-threonine (Thr)/-(Asn-x-Serine (Ser), respectively, wherein X represents an optional amino acid.

3. A colony-stimulating glycoprotein of Claim 1, which had an isoelectric point (pI) of 3.1 - 3.7 as measured by the polyacrylamide gel isoelectric focussing method and the sucrose density-gradient isoelectric focussing method.

4. A colony-stimulating glycoprotein of Claim 1, wherein carbohydrates contained therein are mannose, galactose, N-acetylglucosamine, N-acetylgalactosamine and N-acetylneuramic acid.

5. A colony-stimulating glycoprotein of Claim 1, which has a circular dichroism spectrum having minimum peaks at 208 nm and 222 nm revealing $\alpha$-helix structure.

6. A colony-stimulating glycoprotein of Claim 1, the biological activity of which is not lost by heating to 60 ± 0.5°C for 60 minutes.

7. A colony-stimulating glycoprotein of Claim 1, the infrared spectrum of which is as shown in the accompanying Fig. 3.

8. A colony-stimulating glycoprotein of Claim 1, wherein the 122nd and 140th asparagines in said amino acid sequences have N-glycosidic linkage sites represented by asparagine (Asn)-X-threonine (Thr)/-(Asn-x-Serine (Ser), respectively, wherein X represents an optional amino acid; carbohydrates contained therein are mannose galactose, N-acetylglucosamine, N-acetylgalactosamine and N-acetylneuramic acid; and which has an isoelectric point (pI) of 3.1 - 3.7 as measured by the polyacrylamide gel isoelectric focussing method and the sucrose density-gradient isoelectric focussing method, a circular dichroism spectrum having minimum peaks at 208 nm and 222 nm revealing an $\alpha$-helix structure, an infrared spectrum as shown in the accompanying Fig. 3, and a heat stability such that it does not lose its biological activity on heating at 60°± 0.5°C for 60 minutes.

9. A process for preparing a colony-stimulating glycoprotein having stimulating action on colony formation of monocyte-macrophage cells of mammals and having a molecular weight of 70,000 - 90,000 daltons as measured by the non-reducing sodium dodecyl sulfate polyacrylamide electrophoresis method, which glycoprotein is a homodimer of two glycopolypeptide subunits, which subunits (a) contain respective amino acid sequences which are the same as one another, (b) are bonded to one another through S-S linkage, and each of which subunits has (c) a molecular weight of 35,000 - 45,000 daltons as measured by the said non-reducing electrophoresis method, and (d) an amino acid sequence represented by the formula:

1
Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

50
-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

100
-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

122
-Phe-Asn-Glu-Thr-Lys-Asn-Leu-Leu-Asp-Lys-Asp-Trp-Asn-Ile-Phe-Ser-Lys-Asn-Cys-Asn-

150
-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

189
-Pro-Ser-Met-Ala-Pro-Val-Ala-Gly-Leu-, or

EP 0 276 551 B1

EP 0 276 551 B1

Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

-Phe-Asn-Glu-Thr-Lys-Asn-Leu-Leu-Asp-Lys-Asp-Trp-Asn-Ile-Phe-Ser-Lys-Asn-Cys-Asn-

-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

-Pro-Ser-Met-Ala-Pro-Val-Ala-Gly-Leu-Thr-Trp-Glu-Asp-Ser-Glu-Gly-Thr-Glu-Gly-Ser-

-Ser-Leu-Leu-Pro-Gly-Glu-Gln-Pro-Leu-His-Thr-Val-Asp-Pro- , or

Glu-Glu-Val-Ser-Glu-Tyr-Cys-Ser-His-Met-Ile-Gly-Ser-Gly-His-Leu-Gln-Ser-Leu-Gln-
-Arg-Leu-Ile-Asp-Ser-Gln-Met-Glu-Thr-Ser-Cys-Gln-Ile-Thr-Phe-Glu-Phe-Val-Asp-Gln-
-Glu-Gln-Leu-Lys-Asp-Pro-Val-Cys-Tyr-Leu-Lys-Lys-Ala-Phe-Leu-Leu-Val-Gln-Asp-Ile- 50
-Met-Glu-Asp-Thr-Met-Arg-Phe-Arg-Asp-Asn-Thr-Pro-Asn-Ala-Ile-Ala-Ile-Val-Gln-Leu- 100
-Gln-Glu-Leu-Ser-Leu-Arg-Leu-Lys-Ser-Cys-Phe-Thr-Lys-Asp-Tyr-Glu-Glu-His-Asp-Lys-
-Ala-Cys-Val-Arg-Thr-Phe-Tyr-Glu-Thr-Pro-Leu-Gln-Leu-Leu-Glu-Lys-Val-Lys-Asn-Val- 140
-Phe-Asn-Glu-Thr-Lys-Asn-Leu-Leu-Asp-Lys-Asp-Trp-Asn-Ile-Phe-Ser-Lys-Asn-Cys-Asn- 122 / 150
-Asn-Ser-Phe-Ala-Glu-Cys-Ser-Ser-Gln-Asp-Val-Val-Thr-Lys-Pro-Asp-Cys-Asn-Cys-Leu-
-Tyr-Pro-Lys-Ala-Ile-Pro-Ser-Ser-Asp-Pro-Ala-Ser-Val-Ser-Pro-His-Gln-Pro-Leu-Ala-
-Pro-Ser-Met-Ala-Pro-Val-Ala-Gly-Leu-Thr-Trp-Glu-Asp-Ser-Glu-Gly-Thr-Glu-Gly-Ser- 189 / 200
-Ser-Leu-Leu-Pro-Gly-Glu-Gln-Pro-Leu-His-Thr-Val-Asp-Pro-Gly-Ser-Ala-Lys-Gln-Arg- 214
-Pro-Pro-Arg-Ser-Thr-Cys-Gln-Ser-Phe-Glu-Pro-Pro-Glu-Thr-Pro-Val-Val-Lys-, 238

which process comprises the steps of (a) collecting a fraction of protein having a molecular weight of 70,000 daltons or more from normal human urine, (b) subjecting the fraction to adsorption treatment by contacting with a hydrophobic affinity material equilibrated with 1 - 4 M salt-containing buffer and to elution treatment with a 0.5 - 1.0 M salt-containing buffer, (c) subjecting the eluate, optionally after concentration thereof, to high-performance liquid gel filtration to recover a fraction containing a component of molecular weight of 70,000 - 150,000 daltons, and (d) subjecting the fraction to reverse phase high-performance liquid chromatography at pH 1-2 to recover the colony-stimulating glycoprotein by a linear gradient concentration method with a solvent containing 0.1% trifluoroacetic acid; the above purification steps (a), (b) and (c) being carried out at a pH of 6.5 - 7.5.

10. A process according to claim 9, wherein the collection of the fraction having a molecular weight of 70,000 daltons or more is carried out by desalting and concentrating the urine by ultrafiltration after clarification and contacting the urine with an anion exchanger and eluting with 0.2 - 0.4 M buffer, and subjecting the elute to gel filtration in a 1 - 4 buffer, these steps being carried out at a pH of 6.5 - 7.5.

11. A process for preparation of a colony-stimulating glycoprotein as defined in claim 9, which comprises isolating and purifying specific antibody against the above colony-stimulating glycoprotein from serum of non-human mammals immunized with the colony-stimulating glycoprotein having stimulating action for formation of colony of monocyte-macrophage cells of mammals, conjugating this specific antibody to an insoluble support to prepare an antibody conjugated support, contacting a solution containing said colony-stimulating glycoprotein with said antibody conjugated support to adsorb the colony-stimulating glycoprotein contained in said solution and eluting the colony-stimulating glycoprotein from said adsorbed antibody conjugated support.

12. A process according to Claim 11, wherein the solution containing colony-stimulating glycoprotein is selected from human urine, culture solution of colony-stimulating glycoprotein producing cells or colony-stimulating factor gene recombined cells and concentrated solution thereof.

13. A process according to Claim 11, wherein the mammals are rabbits, goats, sheep or horses.

14. A process according to Claim 11, 12 or 13, wherein the insoluble support is a cyanogen bromide activated, epoxidized or formylated polysaccharide gel, or an aminoethylated or hydrazidated polymer.

15. A process according to any one of Claims 11 to 14, wherein the contacting is effected so that 5 million - 20 million units of said colony-stimulating glycoprotein contact with 1 g (wet weight) of antibody conjugated support, and the elution is carried out with acetate buffer of pH 2 - 3, 3 - 4 M of thiocyanate or 0.1 - 0.2 M of 2,4-dinitrophenol.

16. A composition having a therapeutic effect on leukopenia, comprising a glycoprotein according to Claim 1 and a physiologically acceptable diluent.

17. A composition according to Claim 16, wherein the glycoprotein according to Claim 1 has been subjected to virus inactivation heat treatment.

**Revendications**

1. Glycoprotéine de stimulation de colonie ayant une action de stimulation sur la formation de colonies de cellules monocytes-macrophages de mammifères et ayant un poids moléculaire de 70 000 - 90 000 daltons, mesuré par la méthode d'électrophorèse sur polyacrylamide au dodécylsulfate de sodium non réductrice, glycoprotéine qui est un homodimère de deux sous-unités glycopolypeptidiques, sous-unités qui (a) sont composées de séquences d'acides aminés correspondantes qui sont identiques l'une à l'autre, (b) sont liées l'une à l'autre par une liaison S-S, et dont chacune des sous-unités (c) a un poids moléculaire de 35 000 - 45 000 daltons mesuré par ladite méthode d'électrophorèse non réductrice, et (d) chacune consiste en la séquence de 214 acides aminés représentée par la formule:

Glu-Glu-Val-Ser-Glu-Tyr-Cys-Ser-His-Met-Ile-Gly-Ser-Gly-His-Leu-Gln-Ser-Leu-Gln-
-Arg-Leu-Ile-Asp-Ser-Gln-Met-Glu-Thr-Ser-Cys-Gln-Ile-Thr-Phe-Glu-Phe-Val-Asp-Gln-
-Glu-Gln-Leu-Lys-Asp-Pro-Val-Cys-Tyr-Leu-Lys-Lys-Ala-Phe-Leu-Leu-Val-Gln-Asp-Ile-
-Met-Glu-Asp-Thr-Met-Arg-Phe-Arg-Asp-Asn-Thr-Pro-Asn-Ala-Ile-Ala-Ile-Val-Gln-Leu-
-Gln-Glu-Leu-Ser-Leu-Arg-Leu-Lys-Ser-Cys-Phe-Thr-Lys-Asp-Tyr-Glu-Glu-His-Asp-Lys-
-Ala-Cys-Val-Arg-Thr-Phe-Tyr-Glu-Thr-Pro-Leu-Gln-Leu-Leu-Glu-Lys-Val-Lys-Asn-Val-
-Phe-Asn-Glu-Thr-Lys-Asn-Leu-Leu-Asp-Lys-Asp-Trp-Asn-Ile-Phe-Ser-Lys-Asn-Cys-Asn-
-Asn-Ser-Phe-Ala-Glu-Cys-Ser-Ser-Gln-Asp-Val-Val-Thr-Lys-Pro-Asp-Cys-Asn-Cys-Leu-
-Tyr-Pro-Lys-Ala-Ile-Pro-Ser-Ser-Asp-Pro-Ala-Ser-Val-Ser-Pro-His-Gln-Pro-Leu-Ala-
-Pro-Ser-Met-Ala-Pro-Val-Ala-Gly-Leu-Thr-Trp-Glu-Asp-Ser-Glu-Gly-Thr-Glu-Gly-Ser-
-Ser-Leu-Leu-Pro-Gly-Glu-Gln-Pro-Leu-His-Thr-Val-Asp-Pro.

**2.** Glycoprotéine de stimulation de colonie selon la revendication 1, dans laquelle les asparagines en positions 122 et 140 dans lesdites séquences d'acides aminés ont des sites de liaison N-glycosidique représentés respectivement par asparagine (Asn)-X-thréonine (Thr)/(Asn)-X-sérine (Ser), où X représente un acide aminé optionnel.

**3.** Glycoprotéine de stimulation de colonie selon la revendication 1, qui a un point isoélectrique (pI) de 3,1

- 3,7, mesuré par la méthode de focalisation isoélectrique sur gel de polyacrylamide et par la méthode de focalisation isoélectrique avec gradient de densité de saccharose.

4. Glycoprotéine de stimulation de colonie selon la revendication 1, dans laquelle les glucides qu'elle contient sont le mannose, le galactose, la N-acétylglucosamine, la N-acétylgalactosamine et l'acide N-acétylneuramique.

5. Glycoprotéine de stimulation de colonie selon la revendication 1, qui a un spectre de dichroïsme circulaire ayant des pics minimums à 208 nm et 222 nm révélant une structure en hélice α.

6. Glycoprotéine de stimulation de colonie selon la revendication 1, dont l'activité biologique n'est pas perdue par chauffage à 60° ± 0,5° C pendant 60 minutes.

7. Glycoprotéine de stimulation de colonie selon la revendication 1, dont le spectre infrarouge est tel que représenté sur la figure 3 annexée.

8. Glycoprotéine de stimulation de colonie selon la revendication 1, dans laquelle les asparagines en positions 122 et 140 desdites séquences d'acides aminés ont des sites de liaison N-glycosidique représentés respectivement par asparagine (Asn)-X-thréonine (Thr)/(Asn)-X-sérine (Ser), où X représente un acide aminé optionnel; les glucides qu'elle contient sont le mannose, le galactose, la N-acétylglucosamine, la N-acétylgalactosamine et l'acide N-acétylneuramique; et qui a un point isoélectrique (pI) de 3,1 - 3,7 mesuré par la méthode de focalisation isoélectrique sur gel de polyacrylamide et la méthode de focalisation isoélectrique avec gradient de densité de saccharose, un spectre de dichroïsme circulaire ayant des pics minimums à 208 nm et 222 nm révélant une structure en hélice α, un spectre infrarouge tel que représenté sur la figure 3 annexée, et une stabilité thermique telle qu'elle ne perd pas son activité biologique par chauffage à 60° ± 0,5° C pendant 60 minutes.

9. Procédé de préparation d'une glycoprotéine de stimulation de colonie ayant une action de stimulation sur la formation de colonies de cellules monocytes-macrophages de mammifères et ayant un poids moléculaire de 70 000 - 90 000 daltons, mesuré par la méthode d'électrophorèse sur polyacrylamide au dodécylsulfate de sodium non réductrice, glycoprotéine qui est un homodimère de deux sous-unités glycopolypeptidiques, sous-unités qui (a) sont composées de séquences d'acides aminés correspondantes qui sont identiques l'une à l'autre, (b) sont liées l'une a l'autre par une liaison S-S, et dont chacune des sous-unités (c) a un poids moléculaire de 35 000 - 45 000 daltons mesuré par ladite méthode d'électrophorèse non réductrice, et (d) une séquence d'acides aminés représentée par la formule :

1
Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

50
-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

100
-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

122                                                                                            140
-Phe-Asn-Glu-Thr-Lys-Asn-Leu-Leu-Asp-Lys-Asp-Trp-Asn-Ile-Phe-Ser-Lys-Asn-Cys-Asn-

150
-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

189
-Pro-Ser-Met-Ala-Pro-Val-Ala-Gly-Leu-, ou

EP 0 276 551 B1

Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

-Phe-Asn-Glu-Thr-Lys-Asn-Leu-Leu-Asp-Lys-Asp-Trp-Asn-Ile-Phe-Ser-Lys-Asn-Cys-Asn-

-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

-Pro-Ser-Met-Ala-Pro-Val-Ala-Gly-Leu-Thr-Trp-Glu-Asp-Ser-Glu-Gly-Thr-Glu-Gly-Ser-

-Ser-Leu-Leu-Pro-Gly-Glu-Gln-Pro-Leu-His-Thr-Val-Asp-Pro- , ou

Glu-Glu-Val-Ser-Glu-Tyr-Cys-Ser-His-Met-Ile-Gly-Ser-Gly-His-Leu-Gln-Ser-Leu-Gln-
-Arg-Leu-Ile-Asp-Ser-Gln-Met-Glu-Thr-Ser-Cys-Gln-Ile-Thr-Phe-Glu-Phe-Val-Asp-Gln-
-Glu-Gln-Leu-Lys-Asp-Pro-Val-Cys-Tyr-Leu-Lys-Lys-Ala-Phe-Leu-Leu-Val-Gln-Asp-Ile- (50)
-Met-Glu-Asp-Thr-Met-Arg-Phe-Arg-Asp-Asn-Thr-Pro-Asn-Ala-Ile-Ala-Ile-Val-Gln-Leu- (100)
-Gln-Glu-Leu-Ser-Leu-Arg-Leu-Lys-Ser-Cys-Phe-Thr-Lys-Asp-Tyr-Glu-Glu-His-Asp-Lys-
-Ala-Cys-Val-Arg-Thr-Phe-Tyr-Glu-Thr-Pro-Leu-Gln-Leu-Leu-Glu-Lys-Val-Lys-Asn-Val- (122, 140)
-Phe-Asn-Glu-Thr-Lys-Asn-Leu-Leu-Asp-Lys-Asp-Trp-Asn-Ile-Phe-Ser-Lys-Asn-Cys-Asn-
-Asn-Ser-Phe-Ala-Glu-Cys-Ser-Ser-Gln-Asp-Val-Val-Thr-Lys-Pro-Asp-Cys-Asn-Cys-Leu- (150)
-Tyr-Pro-Lys-Ala-Ile-Pro-Ser-Ser-Asp-Pro-Ala-Ser-Val-Ser-Pro-His-Gln-Pro-Leu-Ala-
-Pro-Ser-Met-Ala-Pro-Val-Ala-Gly-Leu-Thr-Trp-Glu-Asp-Ser-Glu-Gly-Thr-Glu-Gly-Ser- (189, 200)
-Ser-Leu-Leu-Pro-Gly-Glu-Gln-Pro-Leu-His-Thr-Val-Asp-Pro-Gly-Ser-Ala-Lys-Gln-Arg- (214)
-Pro-Pro-Arg-Ser-Thr-Cys-Gln-Ser-Phe-Glu-Pro-Glu-Thr-Pro-Val-Val-Lys-, (238)

procédé qui comprend les étapes consistant

(a) à recueillir une fraction de protéine ayant un poids moléculaire de 70 000 daltons ou plus à partir d'urine humaine normale,

(b) à soumettre la fraction à un traitement d'adsorption par contact avec un matériau à affinité hydrophobe équilibré avec un tampon contenant 1 - 4 M de sel et à un traitement d'élution avec un tampon contenant 0,5 - 1,0 M de sel,

(c) à soumettre l'éluat, éventuellement après sa concentration, à une filtration de liquide sur gel sous

33

pression élevée pour recueillir une fraction contenant un composant de poids moléculaire compris entre 70 000 et 150 000 daltons, et

(d) à soumettre la fraction à une chromatographie liquide sous pression élevée à phase inversée à pH 1-2 pour recueillir la glycoprotéine de stimulation de colonie par une méthode de concentration à gradient linéaire avec un solvant contenant 0,1% d'acide trifluoroacétique, les étapes de purification précédentes (a), (b) et (c) étant effectuées à un pH de 6,5 - 7,5.

10. Procédé selon la revendication 9, dans lequel le recueil de la fraction ayant un poids moléculaire de 70 000 daltons ou plus est effectué en dessalant et concentrant l'urine par ultrafiltration après clarification et en mettant l'urine en contact avec un échangeur d'anion et en éluant avec un tampon 0,2-0,4 M, et en soumettant l'éluat à une filtration sur gel dans un tampon 1 - 4 M, ces étapes étant effectuées à un pH de 6,5 - 7,5.

11. Procédé de préparation d'une glycoprotéine de stimulation de colonie selon la revendication 9, procédé qui consiste à isoler et purifier un anticorps spécifique vis-à-vis de la glycoprotéine de stimulation de colonie précédente provenant de sérum de mammifères non humains immunisés avec la glycoprotéine de stimulation de colonie ayant une action de stimulation sur la formation de colonies de cellules monocytes-macrophages de mammifères, à conjuguer cet anticorps spécifique à un support insoluble pour préparer un support conjugué à un anticorps, à mettre une solution contenant ladite glycoprotéine de stimulation de colonie en contact avec ledit support conjugué à un anticorps pour adsorber la glycoprotéine de stimulation de colonie contenue dans ladite solution, et à éluer la glycoprotéine de stimulation de colonie à partir dudit support conjugué à l'anticorps qui l'a adsorbée.

12. Procédé selon la revendication 11, dans lequel la solution contenant de la glycoprotéine de stimulation de colonie est choisie entre l'urine humaine, une solution de culture de cellules productrices de la glycoprotéine de stimulation de colonie ou de cellules recombinées de gène du facteur de stimulation de colonie et leurs solutions concentrées.

13. Procédé selon la revendication 11, dans lequel les mammifères sont les lapins, les chèvres, les moutons ou les chevaux.

14. Procédé selon la revendication 11, 12 ou 13, dans lequel le support insoluble est un gel de polysaccharide activé au bromure de cyanogène, époxydé ou formylé, ou un polymère ayant subi une aminoéthylation ou une hydrazidation.

15. Procédé selon l'une quelconque des revendications 11 à 14, dans lequel la mise en contact est effectuée de telle sorte que 5 millions à 20 millions d'unités de ladite glycoprotéine de stimulation de colonie soient en contact avec 1 g (poids sec) du support conjugué à l'anticorps, et l'élution est effectuée avec un tampon d'acétate de pH 2 - 3, 3 - 4 M de thiocyanate ou 0,1 - 0,2 M de 2,4-dinitrophénol.

16. Composition ayant un effet thérapeutique sur la leucopénie, comprenant une glycoprotéine selon la revendication 1 et un diluant acceptable sur le plan physiologique.

17. Composition selon la revendication 16, dans laquelle la glycoprotéine selon la revendication 1 a été soumise à un traitement thermique d'inactivation de virus.

**Patentansprüche**

1. Kolonie-stimulierendes Glycoprotein, welches eine stimulierende Wirksamkeit auf die Koloniebildung von Monozyte-Makrophagenzellen von Säugern besitzt, und welches ein Molekulargewicht von 70 000 bis 90 000 Dalton, welches durch das nicht-reduzierende Natriumdodecylsulfatpolyacrylamid-Elektrophoreseverfahren gemessen wurde, besitzt, welches Glycoprotein ein Homodimer von zwei Glycopolypeptid-Subeinheiten ist, welche Subeinheiten (a) aus den entsprechenden gleichen Aminosäuresequenzen Zusammengesetzt sind, (b) miteinander durch eine S-S-Bindung verbunden sind, und jede dieser Subeinheiten (c) ein Molekulargewicht von 35 000 bis 45 000 Dalton aufweist, welches durch das genannte nicht-reduzierende Elektrophoreseverfahren gemessen wurde, und (d) jede aus den 214 Aminosäuresequenzen, welche durch die folgende Formel dargestellt sind:

Glu-Glu-Val-Ser-Glu-Tyr-Cys-Ser-His-Met-Ile-Gly-Ser-Gly-His-Leu-Gln-Ser-Leu-Gln-
-Arg-Leu-Ile-Asp-Ser-Gln-Met-Glu-Thr-Ser-Cys-Gln-Ile-Thr-Phe-Glu-Phe-Val-Asp-Gln-
-Glu-Gln-Leu-Lys-Asp-Pro-Val-Cys-Tyr-Leu-Lys-Lys-Ala-Phe-Leu-Leu-Val-Gln-Asp-Ile-
-Met-Glu-Asp-Thr-Met-Arg-Phe-Arg-Asp-Asn-Thr-Pro-Asn-Ala-Ile-Ala-Ile-Val-Gln-Leu-
-Gln-Glu-Leu-Ser-Leu-Arg-Leu-Lys-Ser-Cys-Phe-Thr-Lys-Asp-Tyr-Glu-Glu-His-Asp-Lys- (100)
-Ala-Cys-Val-Arg-Thr-Phe-Tyr-Glu-Thr-Pro-Leu-Gln-Leu-Leu-Glu-Lys-Val-Lys-Asn-Val-
-Phe-Asn-Glu-Thr-Lys-Asn-Leu-Leu-Asp-Lys-Asp-Trp-Asn-Ile-Phe-Ser-Lys-Asn-Cys-Asn- (122 / 150)
-Asn-Ser-Phe-Ala-Glu-Cys-Ser-Ser-Gln-Asp-Val-Val-Thr-Lys-Pro-Asp-Cys-Asn-Cys-Leu-
-Tyr-Pro-Lys-Ala-Ile-Pro-Ser-Ser-Asp-Pro-Ala-Ser-Val-Ser-Pro-His-Gln-Pro-Leu-Ala- (180)
-Pro-Ser-Met-Ala-Pro-Val-Ala-Gly-Leu-Thr-Trp-Glu-Asp-Ser-Glu-Gly-Thr-Glu-Gly-Ser- (200)
-Ser-Leu-Leu-Pro-Gly-Glu-Gln-Pro-Leu-His-Thr-Val-Asp-Pro-X. (214)

besteht.

**2.** Kolonie-stimulierendes Glycoprotein von Anspruch 1, worin die 122. und 140. Asparagine in der Aminosäuresequenz N-Glycosid-Bindungsstellen, welche durch Asparagin (Asn)-X-Threonin (Thr)/(Asn)-X-Serin (Ser), worin X eine fakultative Aminosäure darstellt, dargestellt sind, bedeuten.

**3.** Kolonie-stimulierendes Glycoprotein von Anspruch 1, welches einen isoelektrischen Punkt (pI) von 3,1 bis 3,7 aufweit, welcher durch die Polyacrylamidgel-isoelektrische Fokussierungsmethode und das Sucrose-Dichtegradienten-isoelektrische Fokussierungsverfahren bestimmt wurde.

**4.** Kolonie-stimulierendes Glycoprotein von Anspruch 1, worin die darin enthaltenen Kohlehydrate Mannose, Galactose, N-Acetylglucosamin, N-Acetylgalactosamin und N-Acetylneuraminsäure sind.

**5.** Kolonie-stimulierendes Glycoprotein von Anspruch 1, welches ein zirkulares Dichroismusspektrum aufweist, welches Minimum-Peaks bei 208 und 222 nm, welche eine α-Helix-Struktur bezeichnen, aufweist.

**6.** Kolonie-stimulierendes Glycoprotein von Anspruch 1, dessen biologische Aktivität bei Erhitzen auf 60 ± 0,5 °C für 60 min nicht verloren geht.

**7.** Kolonie-stimulierendes Glycoprotein von Anspruch 1, dessen Infrarotspektrum, wie in der beiliegenden Fig.3 gezeigt ist.

**8.** Kolonie-stimulierendes Glycoprotein von Anspruch 1, worin das 122. und das 140. Asparagin der Aminosäuresequenz N-glycosidische Bindungsstellen,welche durch Asparagin (Asn)-X-Threonin (Thr)/ (Asn)-X-Serin (Ser), worin X eine fakultative Aminosäure darstellt, dargestellt sind, die darin enthaltenen Kohlehydrate Mannose, Galactose, N-Acetylglucosamin, N-Acetylgalactosamin und N-Acetylneuraminsäure sind, und welches einen isoelektrischen Punkt (pI) von 3,1 bis 3,7 aufweist, welcher durch das Polyacrylamidgel-isoelektrische Fokussierungsverfahren und das Sucrose-Dichtegradienten-isoelektrische Fokussierungsverfahren gemessen wird, ein zirkulares Dichroismusspektrum, welches Minimum-Peaks bei 208 und 222 nm, welche die α-Helix-Struktur bezeichnet, aufweist, ein Infrarotspektrum, welches in der beiliegenden Fig.3 gezeigt ist, und eine Temperaturstabilität, so daß es seine biologische Wirksamkeit bei Erhitzen auf 60 ± 0,5 °C für 60 min nicht verliert.

**9.** Verfahren zur Herstellung eines Kolonie-stimulierenden Glycoproteins, welches eine Stimulierungswirksamkeit auf die Koloniebildung von Monozyte-Makrophagenzellen von Säugern besitzt, und welches ein Molekulargewicht von 70 000 bis 90 000 Dalton aufweist, welches mit dem nicht-reduzierenden Natriumdodecylsulfatpolyacrylamid-Elektrophoreseverfahren gemessen wird, welches Glycoprotein ein Homodimer von zwei Glycopolypeptid-Subeinheiten ist, welche Subeinheiten (a) entsprechende gleiche Aminosäuresequenzen enthalten, (b) aneinander durch eine S-S-Bindung gebunden sind, und jede der Subeinheiten (c) ein Molekulargewicht von 35 000 bis 45 000 Dalton aufweist, welches durch das nicht-reduzierende Elektrophoseverfahren gemessen wird, und (d) eine Aminosäuresequenz der folgenden Formel aufweist:

Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

-Phe-Asn-Glu-Thr-Lys-Asn-Leu-Leu-Asp-Lys-Asp-Trp-Asn-Ile-Phe-Ser-Lys-Asn-Cys-Asn-

-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

-Pro-Ser-Met-Ala-Pro-Val-Ala-Gly-Leu-, order

37

EP 0 276 551 B1

1
Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-

50
-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-

100
-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-

122                                                                                    140
-Phe-Asn-Glu-Thr-Lys-Asn-Leu-Leu-Asp-Lys-Asp-Trp-Asn-Ile-Phe-Ser-Lys-Asn-Cys-Asn-

150
-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-

189                                                                      200
-Pro-Ser-Met-Ala-Pro-Val-Ala-Gly-Leu-Thr-Trp-Glu-Asp-Ser-Glu-Gly-Thr-Glu-Gly-Ser-

214
-Ser-Leu-Leu-Pro-Gly-Glu-Gln-Pro-Leu-His-Thr-Val-Asp-Pro- , oder

welches Verfahren die folgenden Schritte umfaßt:

(a) Sammeln einer Proteinfraktion, welche ein Molekulargewicht von 70 000 Dalton oder mehr aufweist, aus menschlichem Urin;

(b) Unterwerfen der Fraktion einer Adsorptionsbehandlung, indem sie mit einem hydrophoben Affinitätsmaterial, welches mit 1 bis 4 M Salz enthaltenden Puffer equilibriert wurde, kontaktiert wird und einer Elutionsbehandlung mit einem 0,5 bis 1 M Salz enthaltenden Puffer

1
Glu-Glu-Val-Ser-Glu-Tyr-Cys-Ser-His-Met-Ile-Gly-Ser-Gly-His-Leu-Gln-Ser-Leu-Gln-

-Arg-Leu-Ile-Asp-Ser-Gln-Met-Glu-Thr-Ser-Cys-Gln-Ile-Thr-Phe-Glu-Phe-Val-Asp-Gln-
                                                '50
-Glu-Gln-Leu-Lys-Asp-Pro-Val-Cys-Tyr-Leu-Lys-Lys-Ala-Phe-Leu-Leu-Val-Gln-Asp-Ile-

-Met-Glu-Asp-Thr-Met-Arg-Phe-Arg-Asp-Asn-Thr-Pro-Asn-Ala-Ile-Ala-Ile-Val-Gln-Leu-
                                                                              100
-Gln-Glu-Leu-Ser-Leu-Arg-Leu-Lys-Ser-Cys-Phe-Thr-Lys-Asp-Tyr-Glu-Glu-His-Asp-Lys-

-Ala-Cys-Val-Arg-Thr-Phe-Tyr-Glu-Thr-Pro-Leu-Gln-Leu-Leu-Glu-Lys-Val-Lys-Asn-Val-
         122                                                                  140
-Phe-Asn-Glu-Thr-Lys-Asn-Leu-Leu-Asp-Lys-Asp-Trp-Asn-Ile-Phe-Ser-Lys-Asn-Cys-Asn-
                                           150
-Asn-Ser-Phe-Ala-Glu-Cys-Ser-Ser-Gln-Asp-Val-Val-Thr-Lys-Pro-Asp-Cys-Asn-Cys-Leu-

-Tyr-Pro-Lys-Ala-Ile-Pro-Ser-Ser-Asp-Pro-Ala-Ser-Val-Ser-Pro-His-Gln-Pro-Leu-Ala-
                                       189                                    200
-Pro-Ser-Met-Ala-Pro-Val-Ala-Gly-Leu-Thr-Trp-Glu-Asp-Ser-Glu-Gly-Thr-Glu-Gly-Ser-
                                                       214
-Ser-Leu-Leu-Pro-Gly-Glu-Gln-Pro-Leu-His-Thr-Val-Asp-Pro-Gly-Ser-Ala-Lys-Gln-Arg-
                                                               238
-Pro-Pro-Arg-Ser-Thr-Cys-Gln-Ser-Phe-Glu-Pro-Pro-Glu-Thr-Pro-Val-Val-Lys-,

39

(c) Unterwerfen des Eluats, gegebenenfalls nach Konzentration desselben, einer HPLC-Gelfiltration, um eine Fraktion, welche ein Komponentenmolekulargewicht von 70 000 bis 150 000 Dalton aufweist, zu gewinnen, und

(d) Unterwerfen der Fraktion einer Umkehrphasen-HPLC bei pH 1 bis 2, um das Kolonie-stimulierende Glycoprotein mit einem linearen Gradienten-Konzentrationsverfahren mit einem Lösungsmittel, welches 0,1% Trifluoressigsäure enthält, zu erhalten,

wobei die obigen Reinigungsschritte (a), (b) und (c) bei einem pH von 6,5 bis 7,5 ausgeführt werden.

10. Verfahren nach Anspruch 9, worin das Sammeln der Fraktion, welche ein Molekulargewicht von 70 000 Dalton oder mehr aufweist, durch Entsalzen und Konzentrieren des Urins durch Ultrafiltration nach Klärung, Kontaktieren des Urins mit einem Anionenaustauscher und Eluieren mit 0,2 bis 0,4M Puffer, und Unterwerfen des Eluats einer Gelfiltration in einem 1 bis 4M Puffer, durchgeführt wird, wobei diese Schritte bei einem pH von 6,5 bis 7,5 durchgeführt werden.

11. Verfahren zur Herstellung eines Kolonie-stimulierenden Glycoproteins nach Anspruch 9, welches Verfahren die folgenden Schritte umfaßt: Isolieren und Reinigen spezifischer Antikörper gegen das oben genannte Kolonie-stimulierende Glycoprotein aus Serum von nicht-menschlichen Tieren, welche mit dem Kolonie-stimulierenden Glycoprotein, welches eine Stimulierungswirkung für die Bildung von Kolonien von Monozyte-Monophagenzellen in Säugern aufweist, immunisiert wurden, Konjugieren dieser spezifischen Antikörper an einen unlöslichen Träger, um einen Antikörper-konjugierten Träger zu bilden, Kontaktieren einer Lösung, welche diese Kolonie-stimulierenden Glycoproteine enthält, mit einem Antikörper-konjugierten Träger, um die Kolonie-stimulierenden Glycoproteine, welche in der genannten Lösung enthalten sind, zu adsorbieren, und Eluieren der Kolonie-stimulierenden Glycoproteine von dem adsorbierten Antikörper-konjugierten Träger.

12. Verfahren nach Anspruch 11, worin die das Kolonie-stimulierende Glycoprotein enthaltende Lösung aus menschlichem Urin, der Kulturlösung von Kolonie-stimulierendem Glycoprotein, produzierenden Zellen oder Kolonie-stimulierenden Faktor-Gen rekombinanten Zellen und konzentrierten Lösungen davon gewonnen wird.

13. Verfahren nach Anspruch 11, worin die Säuger, Kaninchen, Geißen, Schafe oder Pferde sind.

14. Verfahren nach Anspruch 11, 12 oder 13, worin der unlösliche Träger aktiviertes Bromcyan, epoxidiertes oder formyliertes Polysaccharidgel oder ein Amino-ethyliertes oder hydratisiertes Polymer ist.

15. Verfahren nach einem der Ansprüche 11 bis 14, worin das Kontaktieren so durchgeführt wird, daß 5 bis 20 Millionen Einheiten des genannten Kolonie-stimulierenden Glycoproteins mit 1 g (Naßgewicht) Antikörper-konjugierten Träger kontaktiert werden, und die Elution mit einem Acetatpuffer von pH 2 bis 3 in 3 bis 4M Thiocyanat oder 0,1 bis 0,2M 2,4-Dinitrophenol durchgeführt wird.

16. Zusammensetzung, welche eine therapeutische Wirkung auf Leukopenie aufweist, welche ein Glycoprotein nach Anspruch 1 und ein physiologisch verträgliches Verdünnungsmittel aufweist.

17. Zusammensetzung nach Anspruch 16, worin das Glycoprotein nach Anspruch 1 einer Virus-inaktivierenden Hitzebehandlung unterworfen wurde.

# F I G. I

# F I G. 2

EP 0 276 551 B1

F I G. 3